(19) Europäisches Patentamt

European Patent Office

Office européen des brevets

(11) **EP 1 646 863 B1**

(12) **EUROPEAN PATENT SPECIFICATION**

(45) Date of publication and mention
of the grant of the patent:
**02.11.2006 Bulletin 2006/44**

(51) Int Cl.:
*G01N 21/87* (2006.01)   *G01N 33/38* (2006.01)

(21) Application number: **04744939.2**

(22) Date of filing: **06.07.2004**

(86) International application number:
**PCT/IL2004/000599**

(87) International publication number:
**WO 2005/003745 (13.01.2005 Gazette 2005/02)**

(54) **METHOD AND SYSTEM FOR PREDICTING THE COLOR OF GEMSTONES**

VERFAHREN UND VORRICHTUNG ZUR VORAUSBERECHNUNG DER FARBEN VON EDELSTEINEN

PROCEDE ET SYSTEME DE PREDICTION DE LA COULEUR DE PIERRES PRECIEUSES

(84) Designated Contracting States:
**AT BE BG CH CY CZ DE DK EE ES FI FR GB GR HU IE IT LI LU MC NL PL PT RO SE SI SK TR**

(30) Priority: **07.07.2003 IL 15680803**

(43) Date of publication of application:
**19.04.2006 Bulletin 2006/16**

(73) Proprietor: **SARIN COLOR TECHNOLOGIES LTD.**
**52522 Ramat Gan (IL)**

(72) Inventor: **ALTMAN, Gershon**
**Tel Mond 40600 (IL)**

(74) Representative: **Minoja, Fabrizio**
**Bianchetti Bracco Minoja S.r.l.**
**Via Plinio, 63**
**20129 Milano (IT)**

(56) References cited:
• **SCHMIDT W: "Optische Spektroscopie" 2000, WILEY , WEINHEIM , XP002299564 page 123 - page 131**
• **GOLLON P, LEDERER U: "Diamond valuation: The cutting edge" PROFESSIONAL JEWELER MAGAZINE ARCHIVES, [Online] June 2003 (2003-06), XP002299562 Retrieved from the Internet: URL:http: //www.professionaljeweler.com/arc hives/ articles/2003/jun03/0603man2.html> [retrieved on 2004-10-06]**
• **GOLLON P, LEDERER U: PROFESSIONAL JEWELER MAGAZINE ARCHIVES, [Online] May 2003 (2003-05), XP002299563 Retrieved from the Internet: URL:http: //www.professionaljeweler.com/arc hives/ articles/2003/may03/0503man4.html> [retrieved on 2004-10-06]**

## Description

## FIELD OF THE INVENTION

This invention relates to methods and devices of performing color analysis of gemstones.

## BACKGROUND OF THE INVENTION

The visual appearance of a gemstone to the human eye under natural or daylight-approximating light may be a primary indicator of the quality of the gemstone. The latter is known to be assessed by a person's intensive visual comparison of the gemstone's color and clarity with a reference set of master gemstones arranged along a grading scale. With diamonds, one of the most commonly used international grading standards is the Gemological Institute of America's (GIA) "D to Z" scale, which ranges from colorless- to- light yellow. This scale has been calibrated to GIA's set of "master color comparison diamonds" often referred to as "the master stones", and has served as a historical standard with respect to the evaluation of a diamond's color.

Various devices have been developed to automate the evaluation of a gemstone's color and clarity. For example, US 6,473,164 discloses a system for analyzing the color of a diamond comprising a daylight-approximating light source for illuminating the diamond, a light detector for detecting light emerging from the illuminated diamond, and an optical measurement device, such as a spectrophotometer, for measuring characteristics of the light detected by the detector. The system further comprises an optical analysis mechanism, such as a data processor, for comparing measurement data from the optical measurement device to a historical standard and/or for converting the measurement data into CIE color space.

## SUMMARY OF THE INVENTION

The present invention suggests a method of predicting a first color of a cut gemstone having a predetermined first geometry, said cut gemstone to be cut from an uncut gemstone having a second color and a second geometry including:

(a) determining an absorption coefficient of the uncut gemstone's material based on said measured second color and on said measured second geometry, using calculations relating to behavior of light in the uncut gemstone; and
(b) deducing said first color of the cut gemstone based on said predetermined first geometry and the absorption coefficient determined in step (i), using calculations relating to the behavior of light in said cut gemstone.

The method in accordance with the present invention is directed to predicting the color that a gemstone of a chosen geometry will have if and when it is cut from another gemstone. The gemstone of the chosen geometry does not yet exist at the time of the color prediction, but is herein referred to as the 'cut gemstone', while the existing gemstone from which it may be cut and on which the prediction is based, is referred to herein as the 'uncut gemstone'. Thus, in the present description, 'cut' and 'uncut' are not necessarily indicative of the real state nor of previous or subsequent actions performed or to be performed on either gemstone.

In accordance with the present invention, the color prediction of the cut gemstone is based on a measured (e.g. mapped) geometry of the uncut gemstone on its measured color, on pre-determined geometry (map) which the cut gemstone is intended to have, defined in the same terms as the measured geometry of the uncut gemstone, and on calculations of light behavior in both gemstones based on their geometries.

It is known that the color of an object, when it is illuminated by light, may be characterized by the object's transmittance of the light, which may be graphed as a function of wavelength comprised therein, such a graph being representative of the color of the object. The transmittance of the object may be measured by known spectral measurement devices (e.g. a colorimeter, a spectrophotometer). Likewise, it is known to convert the transmittance, such as by use of a table or a processor with a suitable conversion algorithm, to the color of the object that it represents.

Calculations relating to the behavior of light in the uncut and cut gemstones in view of their geometries in the method of the present invention are preferably performed by using known physical principles defining reflection, refraction and absorption of light within a generally transparent body, and explanations are presented below as to how these principles are used in the method of the present invention.

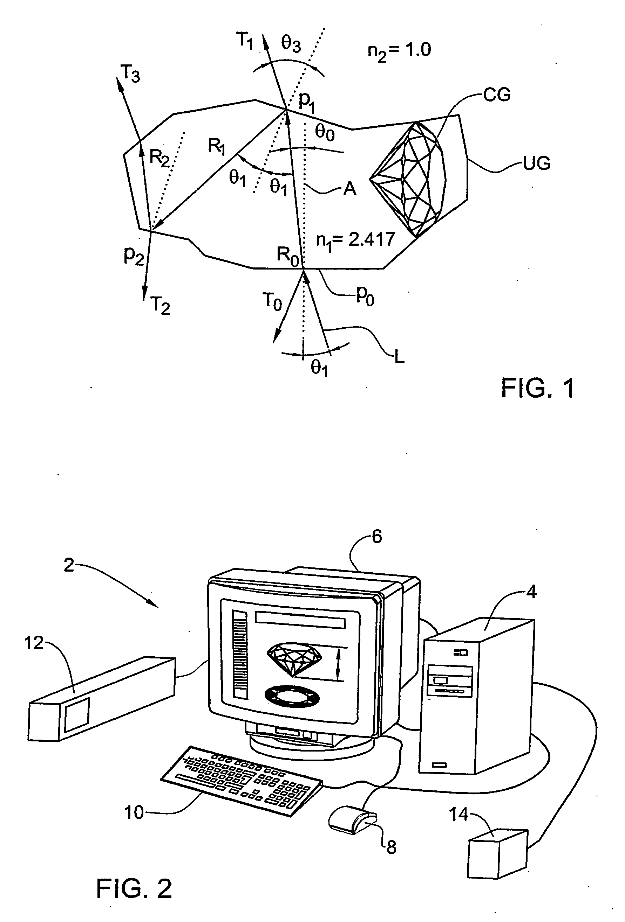

Reference is first made to Fig. 1, which shows a cross-section of an uncut gemstone UG, whose material has an index of refraction $n_1$. The uncut gemstone UG is located within a surrounding medium having an index of refraction $n_2$. In accordance with the laws of reflection and refraction of light passing between media with different refraction indices, a light ray L incident on one of the faces of the uncut gemstone UG with an angle of incidence $\theta_1$ at boundary point $p_0$ thereof between the material and the surrounding medium, splits into a reflected component $T_0$ and a refracted component $R_0$. The component $T_0$ is reflected back into the surrounding medium at an angle equal to the angle of incidence $\theta_l$, while the component $R_0$ enters the material at an angle $\theta_0$ and propagates within the material until it impinges on the next boundary point $p_1$, where $R_0$, in the same manner described above for L, splits into two components: a transmitted component $T_1$, which refracts as it exits the material, and a reflected component $R_1$, which is reflected back into the material with the same angle of incidence

with which $R_0$ impinged boundary point $p_1$. The reflected component $R_1$ continues onto the next boundary point $p_2$, where it splits similarly into transmitted and reflected components $T_2$ and $R_2$, with subsequent reflected components continuing in this manner until the light ray within the material (i.e. the power of the last reflected component) has completely attenuated.

The division of light ray L into components may be quantified by the reflectance and the transmittance of the light ray L by gemstone UG, the former being indicative of the extent of light intensity reflected at the boundary points (i.e. irradiance of components $T_0$, $R_1$, $R_2$, etc.) and the latter being indicative of the extent of light intensity refracted at the boundary points (i.e. irradiance of components $R_0$, $T_1$, $T_2$, etc.). The intensity of the reflected and transmitted components may be determined by energy conservation, which dictates that the irradiance of a ray or component thereof impinging at any boundary point is the sum of the irradiances of the resulting reflected and refracted components emerging from that point.

Thus, calculations of the transmittance and the reflectance of an object for light with known characteristic may be performed based on the indexes of refraction $n_1$ and $n_2$, and the angles of reflection and refraction connected with the object's geometry. For a multitude of initially incident light rays of non-polarized light, the reflected and transmitted light is typically split into parallel and perpendicular components, the exact equations for which are known in the art. It may often be assumed, for the purpose of calculations, that incident non-polarized light contains an equal distribution of all polarizations, in which case the reflected parallel and perpendicular components, as well as the transmitted ones, may be averaged to arrive at a single value therefor.

The attenuation of the irradiance of the light within an object due to absorption of the light energy by the object's material, as the light passes therethrough, depends on an absorption coefficient of the object's material and on the object's geometry. Thus, it is known in the art that as a light ray having a wavelength $\lambda$ progresses through any material from an initial point $x_0$ (e.g. one of the boundary points $p_1$, $p_2$, etc.) to another point $x$ within the material, its irradiance power $I$ will be reduced exponentially according to the following:

$$I(x) = I_0 e^{-\alpha \Delta x}$$

where $\Delta x$ is the distance between $x$ and $x_0$, and $\alpha$ is the absorption coefficient for light of wavelength $\lambda$, which is characteristic of the material medium.

In view of the above, if the attenuation of the irradiance of the light within the object is known, as well as the object's geometry, the material's absorption coefficient can be determined, and vice versa.

In accordance with the method of the present invention, the transmittance (color) and geometry of the uncut gemstone UG can be measured, the reflectance of the same gemstone may be calculated based on the measured geometry, the attenuation of light in the gemstone may be determined based on the measured transmittance and calculated reflectance and, consequently, the absorption coefficient $\alpha$ of the gemstone's material may be deduced.

In other words, the method of the present invention is based on the realization that there exist in a gemstone three parameters, namely transmittance (color), geometry and absorption coefficient such that, if two of the parameters can be measured or determined otherwise, the third one may be deduced based on calculations relating to the behavior of light in the gemstone. For the sake of convenience, the three parameters will hereinafter be referred to as 'color-related parameters'.

Hence, in accordance with the present invention, it is suggested that a three-dimensional map of the uncut gemstone UG be generated by which the necessary geometrical parameters (e.g. angles, distances) may be measured or calculated. In addition, the transmittance (i.e. color) of the gemstone is determined by a spectral measurement device in which a white light beam of known spectra is projected onto the gemstone UG at a predetermined boundary point and angle of incidence. Based on the mapping and transmittance data, which characterizes two color-related parameters of the gemstone, the reflectance and absorption of the light in the uncut gemstone may be calculated, and using the mapping and the absorption data, the third color-related parameter, i.e. the absorption coefficient $\alpha$ of the uncut gemstone, may be deduced. The term "absorption coefficient" in this case means a series of such coefficients for a range of wavelengths at which the transmittance of the uncut gemstone has been measured. To simplify quantification of the attenuation, it is preferable that the index of refraction $n_2$ of the surrounding medium be near unity (e.g. the surroundings being evacuated or occupied by air) so that it does not participate in absorption of the light energy.

In accordance with the method of the present invention, the proposed geometry, by which the cut gemstone is to be cut from the uncut gemstone UG, is chosen based on a map generated in a manner similar to that in which the mapped geometry of the uncut gemstone was measured. Since the cut gemstone will be cut from the uncut gemstone, it will be of the same material and will therefore have the same absorption coefficient $\alpha$, deduced for the uncut gemstone as described above. With the proposed geometry and the absorption coefficient of the cut gemstone (two color-related parameters) being known, calculations may again be performed based on the physical principles governing the light behavior described above to arrive at the expected absorption and reflectance, and consequently at the expected transmittance (third color-related parameter), which characterizes the predicted color of the chosen cut gemstone. Data acquisition in the method of the present invention may be performed by any suitable apparatus known in the art, such as an image capture device for mapping the geometry of the uncut gemstone and a colorimeter for determining the color thereof. Alternatively, a single device may be employed for mapping and determining the color of the uncut gemstone. Such a device may include a processor for performing the necessary calculations for deducing the absorption coefficient and the color of the cut gemstone. Such a processor may be in the form of a computer for controlling the mapping and color determination in addition to performing the calculations. The computer may include a monitor for displaying results of measurements and calculations such as the map of the uncut gemstone, a range of proposed geometries of the cut gemstone from, among which one geometry is to be chosen, the graph of the transmittance of both the uncut and cut gemstone, and the color that the transmittance graph represents, which may be as a grade on any predetermined scale (e.g. the D to Z scale, particularly when the color is of a yellow trend). The computer may also include data entry means adapted to allow a user to direct the method including mapping, color determination, and entry of the choice of the proposed geometry as well as display of results of measurements and calculations. Furthermore, the computer may include a mapping program, as known in the art, for calculating a multitude of possible geometries according to which the gemstone may be cut from the mapped uncut gemstone and determining that having the largest size and/or otherwise most desirable geometry therefrom. The computer may also include a program for directing one or more of the steps of the method of the present invention to determine the color of a plurality of proposed geometries and determine that which would yield the most desirable color. Thus, the computer may be adapted to arrive at and indicate a plurality of options by which the gemstone may be cut, each with its own geometry and color. Furthermore, the computer may be adapted to perform an optimization of such defined criteria and to thereby determine the option constituting the most valuable cut gemstone that may be cut, in terms of both geometry and color.

The method of the present invention may be performed by a computer program having an algorithm adapted to enable a computer to control the necessary components thereof and devices coupled thereto so as to perform the steps of the method. The computer program may be carried in the form of a diskette, CD, or any other such media. Thus, the present invention also suggests a carrier including an algorithm for performing the method according to the present invention.

The present invention further suggests a gemstone color prediction system for predicting a first color of a cut gemstone having a predetermined first geometry, said cut gemstone to be cut from an uncut gemstone having a second color and a second geometry, the system comprising:

(i) a gemstone-mapping device for measuring said second geometry of the uncut gemstone;
(ii) a spectral measurement device for measuring said second color of the second gemstone;
(iii) a processor adapted to determine an absorption coefficient of the uncut gemstone's material, based on data from said gemstone-mapping and said spectral measurement device, using calculations relating to behavior of light in said uncut gemstone, the processor being further adapted to deduce said first color based on said predetermined first geometry and the deduced absorption coefficient, using calculations relating to behavior of light in said cut.

## BRIEF DESCRIPTION OF THE DRAWINGS

In order to understand the invention and to see how it may be carried out in practice, one embodiment will now be described, by way of non-limiting example only, with reference to the accompanying drawings, in which:

Fig. 1 is a schematic cross-sectional view of an uncut gemstone (UG) and a cut gemstone (CG) to be obtained therefrom, shown for the purpose of illustrating the physical principles underlying the method and system of the present invention;
Fig. 2 is a schematic representation of the system according to the present invention;
Fig. 3 shows a computer-generated display of ray-traces performed for use in the method according to the present invention;
Fig. 4 shows a computer-generated display of a measured transmittance of an uncut gemstone and predicted transmittance of a cut gemstone obtained by the method according to the present invention.

## DETAILED DESCRIPTION OF THE INVENTION

Fig. 2 shows schematically a gemstone color prediction system 2 in accordance with the present invention, in which the color prediction is performed in view of three color-related parameters of a gemstone, which are transmittance (color), geometry and absorption coefficient.

The system 2 comprises a processor in the form of a computer 4, a standard monitor 6, mouse 8, and keyboard 10 coupled thereto. The system 2 further comprises an image capture gemstone-mapping device 12 such known as **"DiaExpert™"** manufactured by Sarin Technologies Ltd., Ramat Gan, Israel. The device 12 is adapted to develop a three-dimensional map of a gemstone placed therein with the aid of the processor, which is operating with DiaExpert™, a gemstone-mapping computer program developed also by Sarin Technologies Ltd. Other suitable such programs include DiaMension™, all of Sarin Technologies Ltd. The system also comprises a colorimeter 14 such known as "DC3000" by Sarin Technologies Ltd. adapted to project a beam of white daylight-like light, having a range $\Delta\lambda$ of component wavelengths, onto a gemstone placed therein and to subsequently measure the transmittance of the light through the gemstone's material. The mapping device 12 and the colorimeter 14 are also coupled to the computer 4. The computer is further provided with a program designed to define, based on two color-related parameters of a gemstone, a third color-related parameter thereof by means of calculations relating to light behavior in the gemstone as described above.

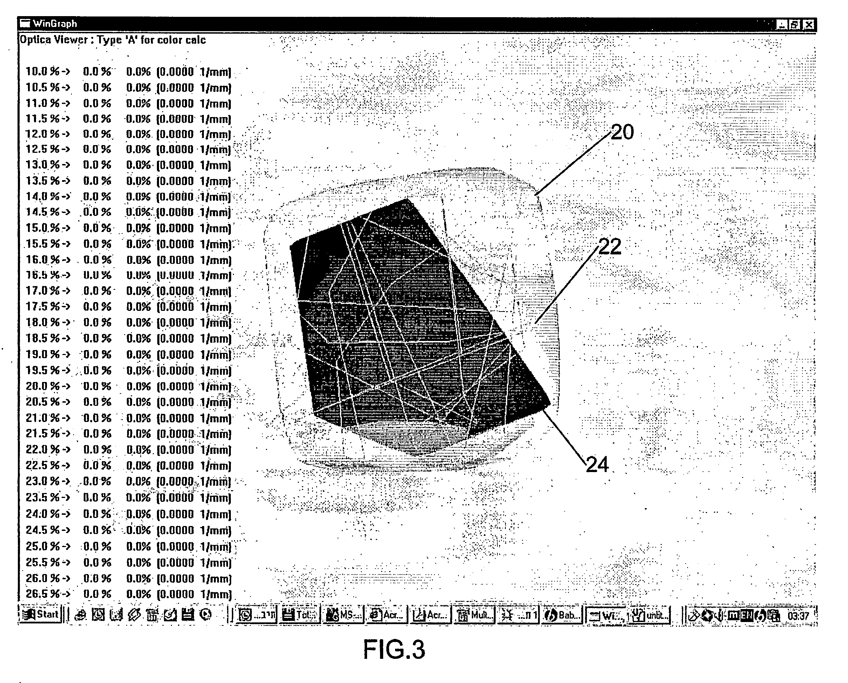

In operation, an uncut gemstone (not shown) is placed within the mapping device 12, which scans the gemstone and a three-dimensional map of the gemstone is developed by DiaExpert™ and subsequently saved by the computer 4. As shown in Fig. 3, DiaExpert™ also allows the mapped uncut gemstone 20 to be displayed on the monitor 6 for viewing. The colorimeter 14 is then calibrated by measuring the spectral distribution of power of the beam while it is still empty (i.e. the power of the beam transmitted through the air therein). The uncut gemstone is then removed from the mapping device 12 and placed into the colorimeter 14, and the beam is projected onto the gemstone at a boundary point and angle of incidence coordinated with the gemstone's map. The ratio between the measured spectral distribution of power transmitted through the gemstone and the spectral distribution of power through the air measured at calibration is known as the "spectral transmittance of the stone", which has herein been designated as simply the transmittance. The measurement and calculation of the transmittance is saved by the computer 4.

As shown in Fig. 3, the computer 4 proceeds to calculate the behavior of light in the uncut gemstone including tracing and, possibly, displaying on the monitor 6 a calculated path 22 along which a monochromatic ray of light would travel when incident on the uncut gemstone at the same locations and incident angles at which the beam was incident on the gemstone in the colorimeter 4 during measurement. The calculations are performed based on the physical principles described above incorporating the reflection, refraction, and attenuation of the ray and using the predetermined values and measured data including the uncut gemstone map 20 and the uncut gemstone's transmittance. The computer 4 then proceeds to deduce a dependency look up table where each of incrementally increasing values of transmittance is matched with a calculated absorption coefficient.

Reverting to Fig. 3, the computer 4 uses DiaExpert™ to proceed to generate a range of proposed geometries for a cut gemstone that may be cut from the mapped uncut gemstone 20. By use of the mouse 8 and/or keyboard 10, a single proposed geometry is chosen from the range, and a virtual map 24 of the allocated cut gemstone is displayed on the monitor 6.

As was done for the uncut gemstone, the computer 4 proceeds to calculate the expected behavior of light in the cut gemstone including tracing and displaying on the monitor 6 a calculated path 22 along which a monochromatic ray of light would travel when incident on the allocated cut gemstone. This tracing uses the same incident point and angle range as later may be projected by colorimeter when measuring the cut gemstone. Since the material of both the uncut and cut gemstones is identical, the absorption coefficient $\alpha$ for each transmittance in the cut gemstone, is now known. Also, the geometry of the cut gemstone has been chosen and the geometrical parameters have also been calculated based thereon. Therefore, the only unknown color-related parameter is the transmittance of the cut gemstone, which the computer 4 proceeds to deduce based on the series of absorption coefficients $\alpha$ found for the uncut gemstone.

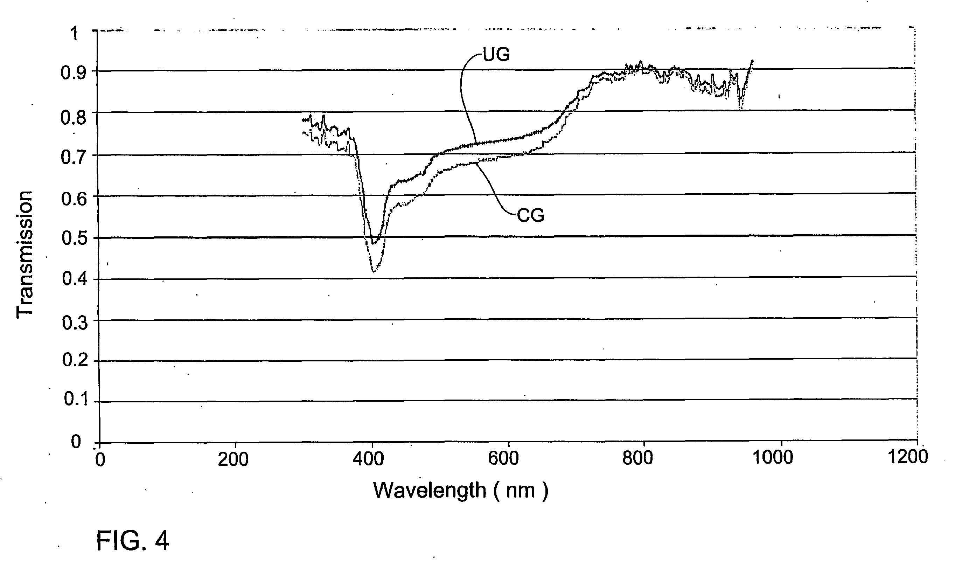

The overall calculation algorithm is thus based on a dependency of transmittance of the cut stone on transmittance values measured on the uncut gemstone and the expected geometry of the cut stone. Using this dependency, the spectral transmittance of the uncut gemstone is converted to the spectral transmittance for the cut stone. The graphs of the transmittance for the gemstones with respect to wavelength over the range $\Delta\lambda$ may be displayed on the monitor 6 separately or together for comparison, as shown, for example, in Fig. 4, where the measured transmittance of the uncut gemstone (UG) is shown with a solid line and the deduced transmittance of the allocated cut gemstone (CG) with a dashed line. Using the new, predicted transmittance for the cut gemstone, the computer 4 may translate the deduced transmittance into the color prediction for the allocated cut gemstone. The predicted color, based on the new spectral transmittance of the cut gemstone, may be indicated on the monitor 6 (by its grade according to any predetermined scale (e.g. the D to Z scale).

It should be understood that the above-described embodiment is only an example of the method and system for predicting the color of a cut gemstone in accordance with the present invention and that the scope of the present invention fully encompasses other embodiments and applications that may become obvious to those skilled in the art. For example, the system described above may have additional devices such as, e.g. a marking or polishing apparatus, grading apparatus for cut, clarity or color grading for performing additional such operations on the uncut gemstone, or a grading device for assessing the color, clarity, and cut grading of the gemstone. Furthermore, the method may be performed and the system embodied in a single apparatus capable of performing all the functions according to the present invention, or alternatively as a plurality of communicating devices, each adapted to perform or constitute a portion thereof.

## Claims

1. A method of predicting a first color of a cut gemstone having a predetermined first geometry, said cut gemstone to be cut from an uncut gemstone having a second color and a second geometry including:

(i) determining an absorption coefficient of the uncut gemstone's material based on said measured second color and said measured second geometry of the uncut gemstone, using calculations relating to behavior of light in said uncut gemstone; and
(ii) deducing said first color of the cut gemstone based on the absorption coefficient determined in step (i), and on said determined first geometry, using calculations relating to the behavior of light in said cut gemstone.

2. A method according to Claim 1, wherein said first and second colors are defined by transmittance.

3. A method according to Claim 1 or 2, wherein said measured second geometry is obtained by generating a measured map of the uncut gemstone and said predetermined first geometry is obtained by generating a calculated map of the cut gemstone.

4. A method according to any one of Claims 1 to 3, wherein said predetermined first geometry is chosen from among a range of possible proposed geometries.

5. A method according to Claim 4 further including:

- defining criteria for an optimal predetermined first geometry from said range based on said first color; and
- choosing the predetermined first geometry from said range based on said criteria.

6. A method according to any one of Claims 1 to 5, wherein said measured second geometry is obtained using a laser gemstone-mapping device.

7. A method according to any one of Claims 1 to 6, wherein said measured second color is obtained by using a spectral measurement device, for example a colorimeter.

8. A method according to any one of Claims 1 to 7, performed by a processor.

9. A method according to any one of Claims 1-8, wherein said deduced first color is provided as a grade in accordance with a predetermined scale.

10. A carrier including a program for performing the method of any one of Claims 1-9.

11. A gemstone color prediction system (2) for predicting a first color of a cut gemstone having a predetermined first geometry, said cut gemstone to be cut, from an uncut gemstone having a second color and a second geometry, the system comprising:

(a) a gemstone-mapping device (12) for measuring said second geometry of the uncut gemstone;
(b) a spectral measurement device (14) for measuring said second color of the uncut gemstone; **characterised by**
(c) a processor (4) adapted to determine an absorption coefficient of the uncut gemstone's material based on data from said gemstone-mapping and said spectral measurement device, and on calculations relating to behavior of light in said uncut gemstone the processor being further adapted to deduce said first color based on said predetermined first geometry, the deduced absorption coefficient and calculations relating to behavior of light in said cut gemstone.

12. A gemstone color prediction system (2) according to Claim 11, wherein said first and second colors are defined by transmittance.

13. A gemstone color prediction system (2) according to Claim 11 or 12, wherein said processor (4) is further adapted to obtain said predetermined first geometry generating a calculated map of the cut gemstone.

14. A gemstone color prediction system (2) according to any one of Claims 11 to 13, wherein said processor (4) is further adapted to provide a range of possible proposed geometries from which said predetermined first geometry is chosen, and preferably even further adapted to provide an optimal predetermined first geometry for choosing from said range in accordance with defined criteria based on said first color.

15. A gemstone color prediction system (2) according to any one of Claims 11 to 14, wherein the (4) processor is further adapted to provide the deduced first color as a grade in accordance with a predetermined scale.

16. A gemstone color prediction system (2) according to any one of Claims 11 to 15, wherein said gemstone-mapping device (12) is adapted to map using a laser.

**Patentansprüche**

1. Verfahren zur Vorhersage einer ersten Farbe eines geschliffenen Schmucksteins mit einer vorbestimmten ersten Geometrie, wobei der geschliffene Schmuckstein aus einem ungeschliffenen Schmuckstein mit einer zweite Farbe und einer zweiten Geometrie geschliffen werden soll, wobei das Verfahren die Schritte umfasst:

(i) Bestimmen eines Absorptionskoeffizienten des Materials des ungeschliffenen Schmucksteins auf der Grundlage der gemessenen zweiten Farbe und der gemessenen zweiten Geometrie des ungeschliffenen Schmucksteins, unter Verwendung von Berechnungen, die sich auf das Verhalten von Licht in dem ungeschliffenen Schmuckstein beziehen; und
(ii) Ableiten der ersten Farbe des geschliffenen Schmucksteins auf der Grundlage des in Schritt (i) bestimmten Absorptionskoeffizienten und der bestimmten ersten Geometrie, unter Verwendung von Berechnungen, die sich auf das Verhalten von Licht in dem geschliffenen Schmuckstein beziehen.

2. Verfahren nach Anspruch 1, wobei die erste und zweite Farbe durch den Lichtdurchlassgrad definiert sind.

3. Verfahren nach Anspruch 1 oder 2, wobei die gemessene zweite Geometrie durch Erzeugen einer durch Messung ermittelten Karte des ungeschliffenen Schmucksteins erhalten wird, und die vorbestimmte erste Geometrie durch Erzeugen einer durch Berechnung ermittelten Karte des geschliffenen Schmucksteins erhalten wird.

4. Verfahren nach einem der Ansprüche 1 bis 3, wobei die vorbestimmte erste Geometrie aus einer Reihe von möglichen vorgeschlagenen Geometrien gewählt wird.

5. Verfahren nach Anspruch 4, des Weiteren umfassend die Schritte:

- Definieren von Kriterien für eine optimale vorbestimmte erste Geometrie aus der Reihe auf der Grundlage der ersten Farbe; und
- Auswählen der vorbestimmten ersten Geometrie aus der Reihe auf der Grundlage der Kriterien.

6. Verfahren nach einem der Ansprüche 1 bis 5, wobei die gemessene zweite Geometrie unter Verwendung einer Lasereinrichtung für die Schmucksteinkartierung erhalten wird.

7. Verfahren nach einem der Ansprüche 1 bis 6, wobei die gemessene zweite Farbe unter Verwendung einer Spektralmesseinrichtung, zum Beispiel eines Kolorimeters, erhalten wird.

8. Verfahren nach einem der Ansprüche 1 bis 7, das von einem Prozessor durchgeführt wird.

9. Verfahren nach einem der Ansprüche 1-8, wobei die abgeleitete erste Farbe als ein Grad der Übereinstimmung mit einer vorbestimmten Skala vorgesehen wird.

10. Träger, der ein Programm umfasst, zur Durchführung des Verfahrens nach einem der Ansprüche 1-9.

11. System (2) zur Vorhersage einer Schmucksteinfarbe zum Vorhersagen einer ersten Farbe eines geschliffenen Schmucksteins mit einer vorbestimmten ersten Geometrie, wobei der geschliffene Schmuckstein aus einem ungeschliffenen Schmuckstein mit einer zweiten Farbe und einer zweiten Geometrie geschliffen werden soll, wobei das System umfasst:

(a) eine Schmuckstein-Kartierungseinrichtung (12) zum Messen der zweiten Geometrie des ungeschliffenen Schmucksteins;
(b) eine Spektralmesseinrichtung (14) zum Messen der zweiten Farbe des ungeschliffenen Schmucksteins; **gekennzeichnet durch**
(c) einen Prozessor (4), der angepasst ist, um einen Absorptionskoeffizienten des Materials des ungeschliffenen Schmucksteins auf der Grundlage von Daten von der Schmuckstein-Kartierungs- und der Spektralmesseinrichtung, und auf der Grundlage von Berechnungen, die sich auf das Verhalten von Licht in dem ungeschliffenen Schmuckstein beziehen, zu bestimmen, wobei der Prozessor des Weiteren angepasst ist, um die erste Farbe auf der Grundlage der vorbestimmten ersten Geometrie, des abgeleiteten Absorptionskoeffizienten und von Berechnungen, die sich auf das Verhalten von Licht in dem geschliffenen Schmuckstein beziehen, abzuleiten.

12. System (2) zur Vorhersage einer Schmucksteinfarbe nach Anspruch 11, wobei die erste und zweite Farbe durch den Lichtdurchlassgrad definiert sind.

13. System (2) zur Vorhersage einer Schmucksteinfarbe nach Anspruch 11 oder 12, wobei der Prozessor (4) des Weiteren angepasst ist, um die vorbestimmte erste Geometrie durch Erzeugen einer durch Berechnung ermittelten Karte des geschliffenen Schmucksteins zu erhalten.

14. System (2) zur Vorhersage einer Schmucksteinfarbe nach einem der Ansprüche 11 bis 13, wobei der Prozessor (4) des Weiteren angepasst ist, um eine Reihe von möglichen vorgeschlagenen Geometrien zu liefern, aus welchen die vorbestimmte erste Geometrie ausgewählt wird, und vorzugsweise noch weiter angepasst ist, um eine optimale vorbestimmte erste Geometrie für die Auswahl aus der Reihe in Übereinstimmung mit definierten Kriterien, die auf der ersten Farbe basieren, zu liefern.

**15.** System (2) zur Vorhersage einer Schmucksteinfarbe nach einem der Ansprüche 11 bis 14, wobei der Prozessor (4) des Weiteren angepasst ist, die abgeleitete erste Farbe als einen Grad der Übereinstimmung mit einer vorbestimmten Skala zu liefern.

**16.** System (2) zur Vorhersage einer Schmucksteinfarbe nach einem der Ansprüche 11 bis 15, wobei die Schmuckstein-Kartierungseinrichtung (12) angepasst ist, die Kartierung unter Verwendung eines Lasers vorzunehmen.

**Revendications**

**1.** Procédé de prédiction d'une première couleur d'une pierre précieuse coupée ayant une première géométrie prédéterminée, ladite pierre précieuse coupée devant être coupée dans une pierre précieuse non coupée ayant une seconde couleur et une seconde géométrie, incluant :

(i) la détermination du coefficient d'absorption du matériau de pierre précieuse non coupée sur la base de ladite seconde couleur mesurée et de ladite seconde géométrie mesurée de la pierre précieuse non coupée, en utilisant des calculs ayant trait au comportement de la lumière dans ladite pierre précieuse non coupée ; et
(ii) la déduction de ladite première couleur de la pierre précieuse coupée sur la base du coefficient d'absorption déterminé dans l'étape (i), et de ladite première géométrie déterminée, en utilisant des calculs ayant trait au comportement de la lumière dans ladite pierre précieuse coupée.

**2.** Procédé selon la revendication 1, dans lequel lesdites première et seconde couleurs sont définies par transmittance.

**3.** Procédé selon la revendication 1 ou 2, dans lequel ladite seconde géométrie mesurée est obtenue en générant une carte mesurée de la pierre précieuse non coupée et ladite première géométrie prédéterminée est obtenue en générant une carte calculée de la pierre précieuse coupée.

**4.** Procédé selon l'une quelconque des revendications 1 à 3, dans lequel ladite première géométrie prédéterminée est choisie dans une gamme de géométries proposées possibles.

**5.** Procédé selon la revendication 4, incluant en outre :

- la définition de critères pour une première géométrie prédéterminée optimale à partir de ladite gamme sur la base de ladite première couleur ; et
- le choix de la première géométrie prédéterminée dans ladite gamme sur la base desdits critères.

**6.** Procédé selon l'une quelconque des revendications 1 à 5, dans lequel ladite seconde géométrie mesurée est obtenue en utilisant un dispositif de cartographie de pierre précieuse par laser.

**7.** Procédé selon l'une quelconque des revendications 1 à 6, dans lequel ladite seconde couleur mesurée est obtenue en utilisant un dispositif de mesure spectrale, par exemple un colorimètre.

**8.** Procédé selon l'une quelconque des revendications 1 à 7, mis en oeuvre par un processeur.

**9.** Procédé selon l'une quelconque des revendications 1 à 8, dans lequel ladite première couleur déduite est fournie sous la forme d'un grade d'après une échelle prédéterminée.

**10.** Support incluant un programme pour mettre en oeuvre le procédé selon l'une quelconque des revendications 1 à 9.

**11.** Système de prédiction de couleur d'une pierre précieuse (2) pour prédire une première couleur d'une pierre précieuse coupée ayant une première géométrie prédéterminée, ladite pierre précieuse coupée devant être coupée dans une pierre précieuse non coupée ayant une seconde couleur et une seconde géométrie, le système comprenant :

(a) un dispositif de cartographie de pierre précieuse (12) pour mesurer ladite seconde géométrie de la pierre précieuse non coupée ;
(b) un dispositif de mesure spectrale (14) pour mesurer ladite seconde couleur de la pierre précieuse non coupée ; **caractérisé par**
(c) un processeur (4) adapté pour déterminer le coefficient d'absorption du matériau de la pierre précieuse non coupée sur la base de données de ladite cartographie de pierre précieuse et dudit dispositif de mesure spectrale, et de calculs ayant trait au comportement de la lumière dans ladite pierre précieuse non coupée, le processeur étant en outre adapté pour déduire ladite première couleur sur la base de ladite première géométrie prédéterminée, du coefficient d'absorption déduit et des calculs ayant trait au comportement de la lumière dans ladite pierre précieuse coupée.

**12.** Système de prédiction de couleur d'une pierre précieuse (2) selon la revendication 11, dans lequel lesdites première et seconde couleurs sont définies par transmittance.

**13.** Système de prédiction de couleur d'une pierre précieuse (2) selon la revendication 11 ou 12, dans lequel ledit processeur (4) est en outre adapté pour obtenir ladite première géométrie prédéterminée générant une carte calculée de la pierre précieuse coupée.

**14.** Système de prédiction de couleur d'une pierre précieuse (2) selon l'une quelconque des revendications 11 à 13, dans lequel ledit processeur (4) est en outre adapté pour fournir une gamme de géométries proposées possibles dans laquelle ladite première géométrie prédéterminée est choisie, et de préférence encore adapté en outre pour fournir une première géométrie prédéterminée optimale pour choisir dans ladite gamme d'après des critères définis sur la base de ladite première couleur.

**15.** Système de prédiction de couleur d'une pierre précieuse (2) selon l'une quelconque des revendications 11 à 14, dans lequel le processeur (4) est en outre adapté pour fournir la première couleur déduite en tant que grade d'après une échelle prédéterminée.

**16.** Système de prédiction de couleur d'une pierre précieuse (2) selon l'une quelconque des revendications 11 à 15, dans lequel ledit dispositif de cartographie de pierre précieuse (12) est adapté pour cartographier en utilisant un laser.

FIG. 1

FIG. 2

FIG.3

FIG. 4